Europäisches Patentamt

European Patent Office

Office européen des brevets

(19)

(11) **EP 0 741 308 A1**

(12) # DEMANDE DE BREVET EUROPEEN

(43) Date de publication:
**06.11.1996 Bulletin 1996/45**

(51) Int Cl.$^6$: **G01T 1/29**, G01T 1/164

(21) Numéro de dépôt: **96400934.4**

(22) Date de dépôt: **02.05.1996**

(84) Etats contractants désignés:
**DE GB NL**

(30) Priorité: **04.05.1995 FR 9505342**

(71) Demandeur: **COMMISSARIAT A L'ENERGIE ATOMIQUE**
**75015 Paris Cédex 15 (FR)**

(72) Inventeurs:
- **Guillemaud, Régis**
  **38000 Grenoble (FR)**
- **Grangeat, Pierre**
  **38330 Saint-Ismier (FR)**

(74) Mandataire: **Dubois-Chabert, Guy et al**
**c/o BREVATOME**
**25, rue de Ponthieu**
**75008 Paris (FR)**

(54) **Procédé de réalisation de la cartographie d'émission d'un corps corrigée de l'atténuation par ce corps**

(57) L'invention concerne un procédé de réalisation d'une cartographie d'émission de rayonnements d'un corps (2) corrigée de l'atténuation des rayonnements par ledit corps, dans lequel une source de transmission de rayonnements (3), apte à prendre plusieurs positions par rapport au corps, envoie des photons vers ledit corps, caractérisé en ce qu'il consiste :

- par chaque position de la source de rayonnements, à effectuer avec un moyen de détection (7) une acquisition d'une mesure en transmission (N) des photons émis par la source de rayonnements et transmis par le corps et une acquisition (E1) d'une projection de mesure en émission des photons émis par le corps, l'acquisition de mesure en transmission et l'acquisition de la projection de mesure en émission étant effectuées avec une même géométrie ;

- à déterminer, pour chaque position de la source de rayonnements, le coefficient de correction de l'atténuation (E4) des rayonnements due au corps pour corriger la projection de mesure en émission (E6) ; et

- à construire une carte d'émission (E2) à partir des projections de mesures en émission corrigées de l'atténuation.

Application au contrôle non destructif et à l'imagerie médicale.

FIG. 3

EP 0 741 308 A1

**Description**

Domaine de l'invention

L'invention concerne un procédé pour réaliser une cartographie d'émission des rayonnements d'un corps, corrigée des atténuations dues à ce même corps.

Elle trouve des applications dans tous les domaines nécessitant la connaissance d'une cartographie réelle de l'émission d'un objet et, en particulier, dans le domaine de l'imagerie médicale, notamment, pour l'étude des différents troubles cardiaques et dans le domaine du contrôle non destructif, notamment, pour le contrôle des déchets radioactifs.

Etat de la technique

Pour la réalisation de cartographies d'émission d'un objet ou d'un corps, il est connu d'utiliser des systèmes tomographiques tridimensionnels (3D), appelés aussi tomographes (3D), permettant de faire des acquisitions bidimensionnelles (2D) des mesures de rayonnements émis par l'objet lui-même et des mesures de rayonnements transmis par une source de rayonnements externe à travers l'objet, suivant une succession d'incidences autour de l'objet et avec une géométrie quelconque.

Une cartographie d'émission caractérise, en chaque point, la valeur locale de l'activité d'émission du rayonnement. La durée de l'émission dépend de la nature chimique de l'élément émetteur : dans le cas de déchets radioactifs, la durée de décroissance de l'émission est généralement longue ; dans le cas de l'imagerie médicale, on injecte au patient une molécule munie d'un traceur radioactif qui est véhiculé par le sang jusqu'à l'organe à examiner. Il y a alors émission de photons par cet organe. Dans ce cas, les isotopes émetteurs ont une période d'émission courte, de l'ordre de quelques heures.

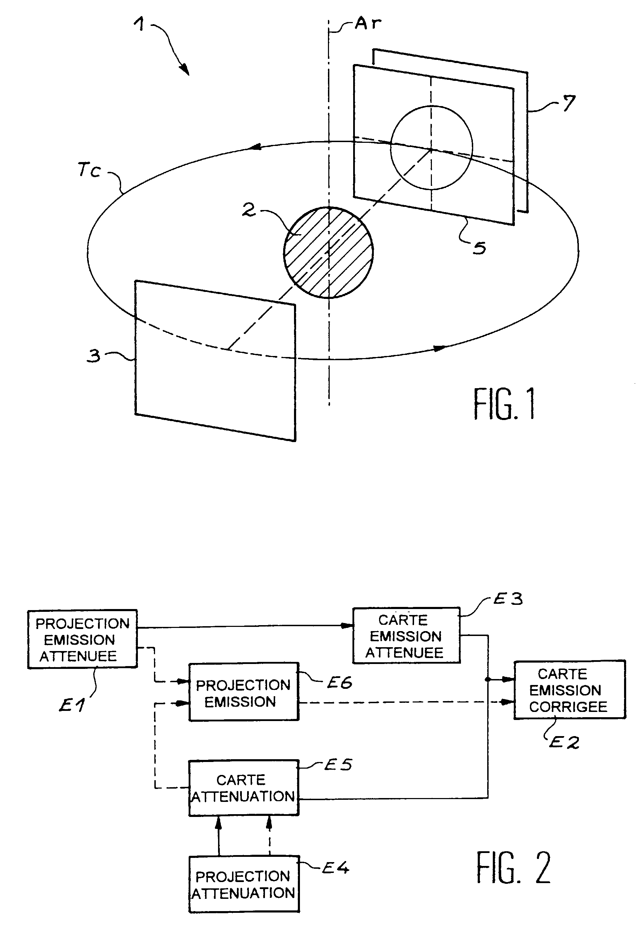

Sur la figure 1, on a représenté un système tomographique 3D tel qu'utilisé habituellement pour la réalisation de cartographies d'émission d'un objet et des cartographies d'atténuation. Ce système tomographique, référencé 1, comporte :

- une source de rayonnements 3 apte à envoyer des photons vers l'objet 2 à étudier ;
- un collimateur 5 aligné avec la source de rayonnements 3 mais opposé par rapport à l'objet 2 ; et
- un moyen de détection 7 placé contre le collimateur 5. Ce moyen de détection 7 peut être une gamma caméra munie de plusieurs détecteurs.

La source de rayonnements 3 est apte à prendre plusieurs positions par rapport à l'objet 2, positions situées sur une trajectoire circulaire Tc. Cette trajectoire Tc se situe dans un plan transaxial qui est le plan perpendiculaire à l'axe de rotation Ar. L'ensemble collimateur/moyen de détection subit les mêmes mouvements de rotation que la source de rayonnements 3 sur la trajectoire Tc.

Sur la figure 1, on a représenté l'axe de rotation Ar passant par le centre de l'objet 2. Il peut toutefois être décentré par rapport audit objet 2.

Plus précisément, le collimateur 5 consiste généralement en une plaque de plomb percée de trous définissant chacun un cône d'acquisition du rayonnement mesuré en un point du moyen de détection 7 et ayant chacun un axe définissant un support de projection intégrale (ou droite d'intégration). C'est ce collimateur 5 qui définit, dans le système tomographique 1, la géométrie de l'acquisition des mesures. Cette géométrie peut être une géométrie parallèle, une géométrie conique, une géométrie éventail ou toute autre géométrie connue de l'homme de l'art.

La source de transmission 3 peut prendre des formes différentes en fonction de la géométrie d'acquisition choisie ; elle peut être un point source situé au point focal d'un collimateur conique, un plan source ou encore une ligne source. Dans le système représenté sur la figure 1, la géométrie d'acquisition étant une géométrie parallèle, la source de rayonnement 3 est un plan source ; pour une telle géométrie parallèle, la source de rayonnement 3 pourrait également être une ligne source ayant un déplacement selon un plan de façon à simuler un plan de transmission.

Un système tomographique tel que celui de la figure 1 avec une ligne source comme source de rayonnements est décrit dans le document D1 : "A scanning line source fot simultaneous emission and transmission measurements in SPECT" de TAN P., 1993, JNM, 34(10), 1 752-1 760.

Un tel système tomographique 3D permet de mettre en oeuvre plusieurs procédés de réalisation d'une cartographie d'émission. Le procédé classique le plus simple consiste en l'acquisition de projections de mesures en émission des rayonnements émis par l'objet, puis en la reconstruction de la cartographie d'émission correspondante.

La reconstruction 3D de ces cartographies peut se faire par des méthodes analytiques, ou des méthodes algébriques itératives, selon la géométrie d'acquisition choisie.

Pour une géométrie parallèle ou une géométrie éventail, on utilise généralement une méthode analytique telle que celle utilisant un algorithme de filtrage/reprojection, décrite par F. NATERER dans le document D2 : "The mathematics of computerized tomography", 1986, John WILEY and sons.

Pour une géométrie conique, on peut mettre en oeuvre une méthode analytique directe par filtrage et rétroprojection telle que celle décrite par L. A. FELDKAMP, L. C. DAVID et J. W. KRESS dans le document D3 : " Pratical cone-beam algorithm", 1984, J. Opt. Soc. Am., 1(6), 612-619.

Pour une telle géométrie, il est possible également de mettre en oeuvre une méthode analytique directe par réarrangement dans le domaine de radon, décrite notamment par B. D. SMITH dans le document D4 : "Image reconstruction from cone-beam projections : necessary and sufficient conditions and reconstruction methods", IEEE Trans. on Med. Imag., MI-4(1), 14-25 et par P. GRANGEAT dans la demande de brevet EP-A-0 292 402.

Quelle que soit la géométrie choisie, des méthodes algébriques itératives peuvent être mises en oeuvre. Elles reposent sur une description de la relation liant l'image à reconstruire aux données mesurées par un système d'équations linéaires et sur une résolution de ce système par itération. Par exemple, une telle méthode est décrite par G. T. GULLBERG, G. L. ZENG, B. M. W. TSUI et J. T. HAGIUS dans le document D5 : "An iterative reconstruction algorithm for single photon emission computed tomography with cone-beam geometry", 1989, Int. J. of Imag. Sys. and Techn. 1, 169-186.

Toutefois, une telle méthode itérative nécessite un important nombre de calculs, ce qui rend le procédé long et fastidieux.

De plus, il est à noter que lorsqu'un objet émet des rayonnements, ces rayonnements sont eux-mêmes atténués par l'objet. En conséquence, la cartographie obtenue est une cartographie d'émission atténuée.

Des procédés, mis en oeuvre par le système tomographique décrit précédemment, ont pour but de corriger la cartographie d'émission de l'atténuation due à l'objet.

L'un de ces procédés utilise, pour l'opération de reconstruction 3D de la cartographie d'émission corrigée, un modèle de cartographie d'atténuation uniforme. Un tel procédé est décrit par S. BELLINI, M. PIACENTINI, C. CAFFORIO et F. ROCCA dans le document D6 : "Compensation of tissue absorption in emission tomography", 1979, IEEE Trans. Acoustics, Speech and Signal Processing, 27(3), 213-218. Cependant, ce procédé offre une cartographie correcte uniquement lorsque la carte d'atténuation n'est pas trop complexe. Par exemple, dans le domaine de l'imagerie médicale, la carte d'atténuation d'un buste avec les poumons, les os et les tissus mous serait beaucoup trop complexe pour permettre une bonne reconstruction de la cartographie d'émission corrigée. Ce procédé ne peut donc être appliqué dans le domaine de l'imagerie médicale cardiaque.

D'autres procédés utilisent, pour la reconstruction 3D de la cartographie d'émission corrigée, une carte d'atténuation réelle. Ces procédés consistent, en plus des acquisitions de projection de mesures en émission, à effectuer des acquisitions de mesures en transmission des rayonnements émis par la source de rayonnements 3 à travers l'objet. Ces acquisitions sont alors prétraitées de manière à en déduire des mesures de projection d'atténuation. Ces procédés consistent ensuite à reconstruire la cartographie de l'atténuation de façon à réaliser, à partir de cette cartographie de l'atténuation et de la cartographie d'émission atténuée, une cartographie d'émission corrigée de l'atténuation.

Ces procédés utilisent donc une carte réelle des mesures en atténuation. Cependant, une telle cartographie d'atténuation fournit des coefficients d'atténuation définis pour l'énergie utilisée pour les mesures en transmission ; elle doit donc être corrigée pour être adaptée à l'énergie correspondant au radioélément utilisé pour les mesures en émission, c'est-à-dire au radioélément se trouvant naturellement dans l'objet (par exemple, déchets radioactifs) ou introduit volontairement dans l'objet ou la personne (notamment en imagerie médicale).

Dans de tels procédés, la correction de l'atténuation dans la cartographie d'émission peut se faire :

a) soit au moyen d'une post-correction du volume d'émission reconstruit à partir des projections atténuées. Dans ce cas, chaque voxel (volume élémentaire) de la carte d'émission est corrigé d'un coefficient lié à l'atténuation moyenne suivant toutes les directions d'acquisition autour du voxel. La correction peut être utilisée une seule fois ou d'une façon itérative par reconstruction-reprojection. Cette méthode est adaptée à une petite source située dans un large volume d'atténuation. Cependant, utilisé dans un cas contraire, elle peut créer une sur-correction au centre de l'objet. Une telle méthode de correction est décrite par L. T. CHANG. dans le document D7 : "A method for attenuation in radionuclide computed tomography", 1978, IEEE Trans. Nulc. Sci., 25(1), 198-123 ;

b) soit par une précorrection des projections d'émission par l'atténuation moyenne calculée sur le même rayon de projection. Cette méthode peut aussi être utilisée de façon itérative. Elle est adaptée à une source d'émission qui a les mêmes contours que le volume d'atténuation correspondant et qui, de plus, est homogène. Cette méthode est décrite par A. MAZET et al. dans le document D8 : "Iterative reconstruction methods for nonuniform attenuation distribution in SPECT", 1993, JNM, 34(7), 1 204-1 209 ;

c) soit par un calcul itératif des coefficients de correction de l'atténuation des projections d'émission. A convergence, ce coefficient pour un pixel du moyen de détection est le rapport de la reprojection calculée à partir du volume d'émission et de la reprojection atténuée calculée à partir des volumes d'émission et d'atténuation. Une telle méthode est décrite par T. MOROZUMI, M. NAKAJIMA, K. OGAWA, S. YUTA dans le document D9 : "Attenuation correction methods using the information of attenuation distribution for single photon emission CT", 1984, Med. Image Tech., 2, 20-28 ;

d) soit par une méthode de reconstruction itérative par maximum de vraisemblance qui corrige l'atténuation en la prenant en compte dans les étages d'estimation et de reprojection. Cette méthode, particulièrement coûteuse en temps de calcul, est décrite par GULLBERG dans le document D5 cité précédemment.

Sur la figure 2, on a représenté par un schéma fonctionnel, deux types de procédés connus, destinés à réaliser une cartographie d'émission corrigée. L'un de ces procédés correspond au parcours montré en traits pointillés. Ce procédé consiste à corriger les projections d'émission atténuées (EI) en projections d'émission corrigée (E6) à partir des informations fournies par une carte d'atténuation réelle (E5) (c'est-à-dire mesurée), construite au moyen des projections d'atténuation (E4). Ce procédé est sensiblement du même type que celui décrit par MAZET dans le document D8 cité précédemment.

L'autre procédé montré sur cette figure 2, correspond au parcours représenté en traits pleins. Ce procédé consiste à construire, d'une part, une carte de l'émission atténuée (E3) et, d'autre part, une carte de l'atténuation (E5) et à déduire de ces deux cartes, une carte d'émission corrigée (E2). Ce procédé est sensiblement du type de celui décrit par CHANG dans le document D7 cité précédemment.

Ces deux procédés, ainsi que tous les autres procédés basés sur l'utilisation d'une cartographie d'atténuation pour corriger la cartographie d'émission sont difficiles à mettre en oeuvre du fait que la cartographie d'atténuation est difficile à obtenir.

En effet, une cartographie d'atténuation peut être calculée au moyen des acquisitions en transmission acquises sur la même gamma caméra que pour l'émission. Dans ce cas précis, les détecteurs de la gamma caméra sont d'une taille suffisante pour acquérir les acquisitions des mesures en émission, mais tronquent souvent les acquisitions des mesures en transmission et, par conséquent, la carte d'atténuation reconstruite comporte des artefacts dus aux troncatures, ce qui nuit à la qualité de la correction.

La cartographie d'atténuation peut être également calculée à partir d'un scanner X qui ne tronque pas les acquisitions des mesures en transmission. Toutefois, dans ce cas, la difficulté se trouve dans le recalage géométrique des données de transmission et d'émission, à la fois en position de repère géométrique, mais aussi à cause de la déformation élastique inévitable de l'objet (ou du patient, dans le cas de l'imagerie médicale) entre les acquisitions d'émission et les acquisitions de transmission.

En outre, ces méthodes à base de cartographie d'atténuation sont coûteuses en temps de calcul car elles nécessitent une reconstruction de la carte d'atténuation, et une reconstruction de la carte d'émission avec correction de l'atténuation généralement itérative.

Exposé de l'invention

L'invention a pour but de remédier aux inconvénients des différents procédés énoncés précédemment. A cette fin, elle propose un procédé pour réaliser une cartographie d'émission corrigée de l'atténuation qui ne nécessite pas la réalisation préalable d'une carte d'atténuation.

De façon plus précise, l'invention concerne un procédé de réalisation d'une cartographie d'émission de rayonnements d'un corps corrigée de l'atténuation des rayonnements par ledit corps, dans lequel une source de transmission de rayonnements, apte à prendre plusieurs positions par rapport au corps, envoie des photons vers ledit corps. Ce procédé se caractérise par le fait qu'il consiste :

- pour chaque position de la source de rayonnements, à effectuer avec un moyen de détection, une acquisition d'une mesure en transmission N des photons émis par la source de rayonnements et transmis par le corps et une acquisition d'une projection de mesure en émission des photons émis par le corps, l'acquisition de mesure en transmission et l'acquisition de la projection de mesure en émission étant effectuées selon une même géométrie ;
- à déterminer, pour chaque position de la source de rayonnements, l'atténuation des rayonnements due au corps pour corriger la projection de mesure en émission ; et
- à construire une carte d'émission à partir des projections de mesures en émission corrigées de l'atténuation.

Selon l'invention, la détermination de l'atténuation consiste :

- à effectuer une acquisition d'une mesure en transmission de référence No obtenue en l'absence du corps ; et
- à déterminer un coefficient de correction C en fonction des acquisitions N et No tel que $Ec = Em \times C$, où $Em$ est la projection de mesure en émission telle que mesurée et $Ec$ est la projection de mesure en émission corrigée de l'atténuation.

Avantageusement, l'acquisition de la mesure en transmission et l'acquisition de la projection de mesure en émission sont effectuées simultanément, les photons émis par la source de transmission de rayonnement et les photons émis par le corps ayant des énergies distinctes.

Dans un mode de réalisation de l'invention dans lequel le corps comporte une zone d'atténuation et une zone d'émission centrées l'une par rapport à l'autre, le coefficient de correction C est approximé par :

$$C \approx \sqrt{\frac{No}{N}},$$

où N est l'acquisition de la mesure en transmission et No est l'acquisition de la mesure en transmission de référence.

Dans un autre mode de réalisation de l'invention, dans lequel le corps comporte une zone d'atténuation de largeur et une zone d'émission décentrée par rapport à ladite zone d'atténuation, le coefficient de correction C est approximé par :

$$C \approx \left(\frac{No}{N}\right)^{Lc/La},$$

où Lc est la distance entre le centre de la zone d'émission et le bord de la zone d'atténuation le plus proche du moyen de détection, N est l'acquisition de la mesure en transmission et No est l'acquisition de la mesure en transmission de référence.

Brève description des figures

- La figure 1, déjà décrite, représente un système tomographique 3D permettant de réaliser des cartographies d'émission corrigée ou non de l'atténuation ;
- la figure 2, déjà décrite, représente un schéma fonctionnel illustrant deux procédés connus pour réaliser des cartographies d'émission corrigées de l'atténuation ;
- la figure 3 représente un schéma fonctionnel illustrant le procédé de l'invention pour réaliser une cartographie d'émission corrigée de l'atténuation sans utiliser de carte d'atténuation ; et
- les figures 4A, 4B et 4C représentent chacune un exemple de fantôme utilisé dans le procédé de l'invention.

Description détaillée de modes de réalisation

Le procédé selon l'invention consiste à réaliser une cartographie de l'émission de rayonnements d'un objet qui est corrigée de l'atténuation de ces rayonnements due à l'objet lui-même.

Selon le domaine dans lequel est appliqué ce procédé, l'objet peut être un corps radioactif ou bien le corps d'un patient dans lequel un radioélément à été introduit ou tout autre type d'objet pouvant émettre naturellement ou non un rayonnement. Dans la suite de la description, on parlera donc indifféremment d'objet ou de corps.

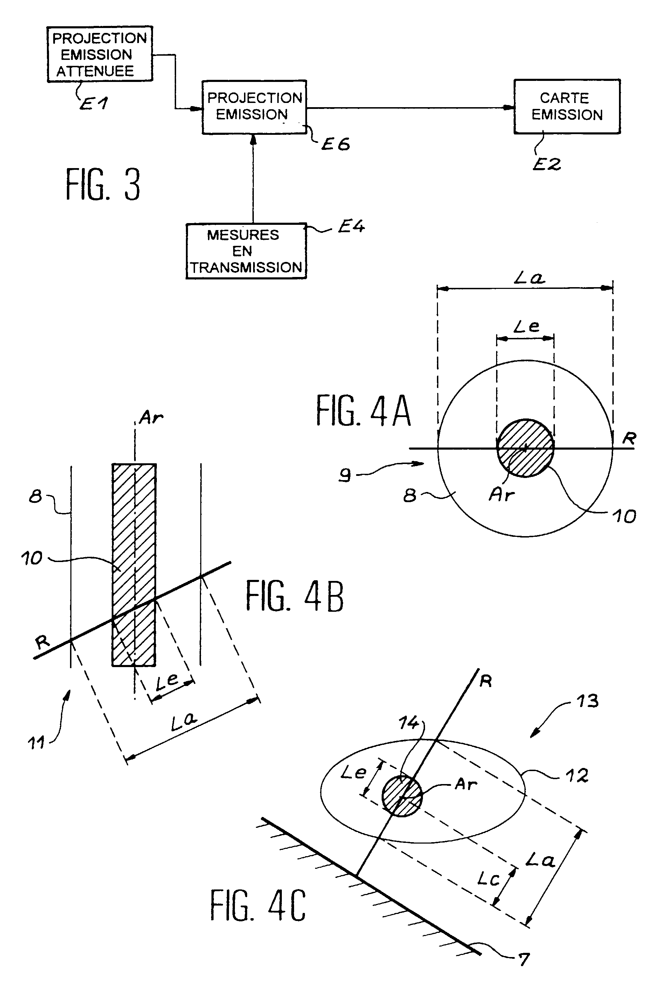

On a représenté sur la figure 3, un schéma fonctionnel illustrant globalement les principales étapes du procédé. Comme on le voit sur cette figure 3, le procédé de l'invention consiste tout d'abord à effectuer l'acquisition des projections de mesures en émission atténuée (étape EI) et, parallèlement, l'acquisition des mesures en transmission. Les mesures de transmission obtenues en E4 sont utilisées pour corriger les projections d'émission atténuée de EI afin d'obtenir des projections d'émission réelle (étape E6). La cartographie d'émission corrigée (étape E2) est alors obtenue par des procédés courants à partir des projections de mesures d'émission réelle, c'est-à-dire corrigées, de l'étape E6.

Selon ce procédé de l'invention, la source de rayonnements 3 est disposée successivement dans plusieurs positions autour du corps ; l'ensemble collimateur/moyen de détection est diamétralement opposé à cette source 3, quelle que soit la position de cette source de rayonnements.

Le procédé consiste, pour chaque position de la source 3, à effectuer une acquisition de la projection de mesure en émission des rayonnements émis par le corps et une acquisition de mesure en transmission des rayonnements émis par la source 3 à travers le corps 2. Ces deux acquisitions sont effectuées pour une même géométrie du système tomographique.

Les acquisitions des mesures en émission et les acquisitions des mesures en transmission peuvent bien sûr être effectuées successivement. Cependant, de façon avantageuse, elles peuvent être faites simultanément, ce qui évite tout risque de déplacement du corps entre deux types d'acquisitions. Dans un tel mode de réalisation, on peut choisir comme source de rayonnements 3, une source émettant des photons d'énergie différente de celle des photons émis par le corps. Le moyen de détection 7 est alors choisi de façon à être capable de séparer les photons émis par le corps 2 des photons émis par la source 3. Cette séparation peut se faire soit par différence d'énergies, soit par des régions d'intérêt glissantes sur le moyen de détection si la source 3 se déplace, soit par tout autre moyen connu de l'homme de l'art.

Par exemple, cette séparation des photons peut se faire au moyen de deux fenêtres d'énergies : l'une des fenêtres étant centrée sur l'énergie des photons d'émission (c'est-à-dire émis par le corps 2), et l'autre fenêtre étant centrée sur l'énergie des photons de transmission (c'est-à-dire émis par la source 3).

On comprendra qu'une acquisition de mesure en transmission est une mesure du nombre de photons détectés sur le moyen de détection 7 après l'atténuation créée par le corps. Cette atténuation est un paramètre physique lié aux matériaux constituant le corps et à la source de rayonnement utilisée. La projection de mesure d'atténuation, ou plus simplement projection d'atténuation, est l'atténuation totale due au corps suivant une droite choisie.

Cette atténuation se détermine au moyen de deux acquisitions de mesure en transmission par le corps (ou simplement, acquisition en transmission) :

- la première acquisition en transmission est une acquisition de référence réalisée lorsque le corps est retiré ; il s'agit en fait d'une acquisition de mesure du rayonnement émis par la source 3. Cette acquisition de référence est notée No ;
- la seconde acquisition en transmission est celle effectuée dans les conditions normales de transmission, décrites précédemment. Cette seconde acquisition, ou acquisition mesurée, est notée N.

L'atténuation $\mu$ est alors déterminée à partir de l'expression :

$$\frac{N}{No} = e^{-X\mu},$$

où No est l'acquisition en transmission de référence, N est l'acquisition en transmission mesurée et $X\mu$ est l'intégrale de l'atténuation $\mu$ suivant une droite choisie.

A partir de cette atténuation $\mu$, on peut déterminer un coefficient de correction C permettant de corriger les projections de mesures en émission de l'atténuation $\mu$. En effet, la projection de mesure en émission corrigée Ec est reliée à la projection de mesure en émission mesurée Em par l'équation :

$$Ec = Em \times C \qquad\qquad (1)$$

avec

$$\begin{cases} Ec = Xf \\ Em = X\mu f \end{cases} \qquad\qquad (2)$$

où Xf est l'intégrale de la mesure en émission suivant une droite, et $X\mu f$ est l'intégrale de la mesure en émission atténuée suivant une droite.

Ce coefficient de correction C dépend donc de la forme et de la position du corps à étudier. Des équivalences ont été déterminées pour C en fonction d'une forme générale du corps, appelée fantôme d'émission.

Dans la suite de la description, on expliquera comment ce coefficient C est déterminé en fonction du type de fantômes correspondant à l'objet ou au corps à étudier.

Le premier fantôme considéré est celui représenté sur la figure 4A ; il s'agit d'un fantôme 9 constitué d'un premier cylindrique 8 dit d'atténuation, et ayant un diamètre La et d'un second cylindrique 10 dit d'émission, qui est de même axe Ar que le cylindre d'atténuation 8 mais de diamètre Le inférieur au diamètre La. Pour un tel fantôme 9, on peut calculer analytiquement le coefficient de correction C en son centre pour un rayon de projection R, perpendiculaire à l'axe du cylindre Ar. Le coefficient C s'écrit alors :

$$C = \frac{\int_{-Le/2}^{Le/2} E.dl}{\int_{-Le/2}^{Le/2} E.e^{-\mu(l+La/2)}.dl} = \frac{\mu.Le.e^{\mu\frac{La}{2}}}{\left(e^{\mu\frac{Le}{2}} - e^{-\mu\frac{Le}{2}}\right)}, \qquad\qquad (3)$$

où $\int E.dl$ correspond à Xf et $\int E.e^{-\mu(1+L-a/2)}dl$ correspond à Xμf, dans le cas particulier de ce premier fantôme.

En effectuant un développement limité de la différence des exponentiels

$$e^{\mu\frac{Le}{2}} - e^{-\mu\frac{Le}{2}}$$

et en remplaçant dans l'équation (3), on obtient un coefficient de correction C de l'ordre de :

$$C \approx \sqrt{e^{\mu.la}} \cdot \left(1 - \frac{(\mu.Le)^2}{24}\right). \qquad (5)$$

Pour une application au domaine de l'imagerie médicale, on peut utiliser un fantôme de ce type, dans lequel le diamètre Le est peu différent du diamètre La, pour approcher une configuration d'acquisitions cérébrales. Un tel fantôme dans lequel le diamètre Le est très faible devant le diamètre La permet d'approcher une configuration d'acquisitions cardiaques avec un coeur émettant petit par rapport au tronc du patient.

Dans ce dernier cas, l'expression de coefficient de correction C peut s'approximer de la même façon que dans le cas précédent :

$$C \approx \sqrt{e^{\mu.La}} \approx \sqrt{\frac{No}{N}}. \qquad (6)$$

Sur la figure 4B, on a représenté un autre exemple de fantôme. Tout comme le fantôme 9 précédent (figure 4A), ce fantôme 11 de la figure 4B comprend un cylindre d'atténuation 8 de diamètre La et un cylindre d'émission 10 de diamètre Le, inférieur à La. Pour ce fantôme, le rayon de projection R passe également par le centre des cylindres. Toutefois, ce rayon de projection R n'est pas perpendiculaire à l'axe Ar du cylindre. Dans ce cas, et lorsque Le « La, le coefficient de correction C s'écrit également :

$$C \approx \sqrt{e^{\mu.La}} \approx \sqrt{\frac{No}{N}}. \qquad (6)$$

De même, dans le cas d'un fantôme de même forme que le fantôme 9, mais pour lequel le rayon de projection R ne passerait pas par l'axe Ar du cylindre, le coefficient de correction C s'écrirait de la même façon que dans l'équation précédente (6).

En conséquence, on comprendra que le coefficient de correction C tel que défini ci-avant est valable en tout point du moyen de détection 7. Les projections de mesures en émission Em peuvent donc être corrigées de la façon suivante :

$$Ec = Em\sqrt{e^{Pa}}$$

ou

$$Ec = Em\sqrt{\frac{No}{N}},$$

où Ec est la projection de mesure en émission corrigée de l'atténuation et Pa est la projection d'atténuation sur le même pixel du moyen de détection, avec Pa = μ.La.

Sur la figure 4C, on a représenté un fantôme 13 comportant une zone d'atténuation 12 et une zone d'émission 14 située à l'intérieur de la zone d'atténuation 12, mais décentrée par rapport à cette dernière. La zone d'atténuation 12 a une longueur La ; la zone d'émission 14 a une longueur Le ; et la longueur suivant le rayon de projection R entre le centre de cette zone d'émission 14 et le bord de la zone d'atténuation 12 la plus proche du moyen de détection 7 est Lc.

Dans ce cas, le coefficient de correction C, pour Le très inférieur à La, s'écrit :

$$C \approx \left( \frac{No}{N} \right)^{Lc/La}. \qquad (7)$$

La projection de mesure en émission corrigée Ec s'écrit en fonction de la projection de mesure en émission Em de la façon suivante :

$$Ec = Em\left( e^{Pa} \right)^{Lc/La}$$

ou

$$Ec = Em\left( \frac{No}{N} \right)^{Lc/La},$$

avec Pa = μ.La.

On comprendra ainsi que les projections en émission sont corrigées de l'atténuation directement à partir des mesures en transmission, sans que la reconstruction d'aucune carte d'atténuation ne soit nécessaire.

En outre, ce procédé peut être mis en oeuvre par un système qui comporte, comme dans l'art antérieur, un moyen de détection 7 permettant de faire l'acquisition de projections non tronquées de l'émission. Cependant, avec ce procédé, les acquisitions en transmission peuvent être tronquées sans que cela nuise à la qualité de la correction puisque les informations de l'acquisition en transmission utiles à la correction sont déduites directement, les informations tronquées n'étant pas utiles à la correction.

Selon un mode de réalisation de l'invention, on utilise une gamma caméra munie d'un système d'autocontourning, c'est-à-dire d'un système permettant d'évaluer ou de mesurer le contour physique de l'objet ou du patient. Ce contour est alors utilisé par la gamma caméra pour faire tourner le moyen de détection 7 le plus près possible de l'objet ou du patient sans toutefois le toucher.

Par exemple, en imagerie cardiaque, on peut utiliser la gamme caméra de SOPHA-MEDICAL® qui possède un tel système d'autocontourning. Dans cet exemple, on positionne un plan source homogène en technétium 99, aligné avec la gamma caméra, mais à l'opposé par rapport au corps ; cette source peut être, par exemple, à 60 cm du corps.

Ce dispositif peut être utilisé pour étudier différents troubles cardiaques. Le protocole utilisé peut être l'injection d'un radio pharmaceutique marqué au thallium 201. Le système tomographique réalise alors une cartographie tridimensionnelle de la répartition du traceur. Dans ce cas, la valeur du coefficient de correction en énergie est :

$$K=1.210.$$

De même que les cartes d'atténuation doivent être corrigées en énergie (comme expliqué précédemment), les coefficients de correction C, définis ci-avant, doivent être corrigés par un facteur multiplicatif K qui est le rapport du coefficient d'atténuation de l'eau à l'énergie utilisée pour la mesure en émission, et du coefficient d'atténuation de l'eau à l'énergie utilisée pour la mesure de transmission, ces coefficients d'atténuation de l'eau étant des grandeurs physiques connues de l'homme de l'art.

La correction de l'atténuation proposée dans cet exemple permet d'étudier la carte d'émission du coeur corrigée des artefacts et des déformations créés par l'atténuation, et donc de faire un meilleur diagnostic. De plus, la correction est rapide et possible même si le détecteur est petit et permet d'acquérir uniquement des acquisitions en transmission tronquées.

## Revendications

1. Procédé de réalisation d'une cartographie d'émission de rayonnements d'un corps (2) corrigée de l'atténuation des rayonnements par ledit corps, dans lequel une source de transmission de rayonnements (3), apte à prendre plusieurs positions par rapport au corps, envoie des photons vers ledit corps, caractérisé en ce qu'il consiste :

   - par chaque position de la source de rayonnements, à effectuer avec un moyen de détection (7) une acquisition d'une mesure en transmission (N) des photons émis par la source de rayonnements et transmis par le corps et une acquisition (EI) d'une projection de mesure en émission des photons émis par le corps, l'acquisition de mesure en transmission et l'acquisition de la projection de mesure en émission étant effectuées avec une même géométrie ;

- à déterminer, pour chaque position de la source de rayonnements, l'atténuation (E4) des rayonnements due au corps pour corriger la projection de mesure en émission (E6) ; et
- à construire une carte d'émission (E2) à partir des projections de mesures en émission corrigées de l'atténuation.

2. Procédé selon la revendication 1, caractérisé en ce que la détermination de l'atténuation comprend une opération de détermination d'un coefficient de correction de l'atténuation consistant :

- à effectuer une acquisition d'une mesure en transmission de référence (No) obtenue en l'absence du corps ; et
- à déterminer un coefficient de correction C en fonction des acquisitions N et No tel que Ec = Em x C, où Em est la projection de mesure en émission telle que mesurée et Ec est la projection de mesure en émission corrigée de l'atténuation.

3. Procédé selon la revendication 1 ou 2, caractérisé en ce que l'acquisition de la mesure en transmission et l'acquisition de la projection de mesure en émission sont effectuées simultanément, les photons émis par la source de transmission de rayonnement et les photons émis par le corps ayant des énergies distinctes.

4. Procédé selon la revendication 2 ou 3, caractérisé en ce que le corps est modélisé par une zone d'atténuation (8) et une zone d'émission (10) centrées l'une par rapport à l'autre, le coefficient de correction (C) est approximé par :

$$C \approx \sqrt{\frac{No}{N}},$$

où N est l'acquisition de la mesure en transmission et No est l'acquisition de la mesure en transmission de référence.

5. Procédé selon la revendication 2 ou 3, caractérisé en ce que, suivant une direction de la mesure d'émission, le corps est modélisé par une zone d'atténuation (12) de largeur (La) et une zone d'émission (14) décentrée par rapport à ladite zone d'atténuation, le coefficient de correction (C) est approximé, suivant cette direction, par :

$$C \approx \left(\frac{No}{N}\right)^{Lc/La},$$

où Lc est la distance entre le centre de la zone d'émission (14) et le bord de la zone d'atténuation (12) le plus proche du moyen de détection, N est l'acquisition de la mesure en transmission et No est l'acquisition de la mesure en transmission de référence.

FIG.1

FIG. 2

FIG. 3

FIG. 4A

FIG. 4B

FIG. 4C

Office européen
des brevets

**RAPPORT DE RECHERCHE EUROPEENNE**

Numero de la demande

EP 96 40 0934

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| Y | WO-A-91 02265 (SOPHA MEDICAL ;COMMISSARIAT ENERGIE ATOMIQUE (FR)) 21 Février 1991<br>* abrégé *<br>* page 7, ligne 16 - page 8, ligne 3 *<br>* page 9, ligne 7 - page 15, ligne 24 *<br>* figures *<br>--- | 1-5 | G01T1/29<br>G01T1/164 |
| Y | THE JOURNAL OF NUCLEAR MEDICINE,<br>vol. 28, no. 5, Mai 1987, NEW YORK USA,<br>pages 844-851, XP002010186<br>BAILEY,D L ET AL.: "improved spect using simultaneous emission and transmission tomography"<br>* en entier*<br>--- | 1-5 | |
| Y | EP-A-0 526 970 (PICKER INTERNATIONAL INC.) 10 Février 1993<br>* abrégé *<br>* page 2, ligne 22 - ligne 45 *<br>* page 4, ligne 28 - page 5, ligne 17 *<br>* page 5, ligne 51 - page 7, ligne 23 *<br>* figures *<br>--- | 1-5 | |
| A | EP-A-0 145 998 (GEN ELECTRIC) 26 Juin 1985<br>* page 4, ligne 1 - page 8, ligne 8 *<br>* figures *<br>--- | 1-3 | DOMAINES TECHNIQUES RECHERCHES (Int.Cl.6)<br>G01T |
| A | WO-A-94 00779 (SIEMENS GAMMASONICS) 6 Janvier 1994<br>* page 3, ligne 15 - page 4, ligne 4 *<br>* page 6, ligne 1 - page 9, ligne 6 *<br>* figures *<br>----- | 1,2 | |

Le présent rapport a été établi pour toutes les revendications

| Lieu de la recherche | Date d'achèvement de la recherche | Examinateur |
|---|---|---|
| LA HAYE | 6 Août 1996 | Datta, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)